# EUROPEAN PATENT SPECIFICATION

(11) **EP 2 356 996 B1**
(45) Date of publication and mention of the grant of the patent: **26.06.2013**
(21) Application number: 10012342.1
(22) Date of filing: 03.05.2004
(51) Int. Cl.: A61K 38/17, A61K 39/395, A61K 47/48, A61P 25/28

(54) **Treatment for Alzheimer's disease**
Behandlung von Alzheimerkrankheit
Traitement de la maladie d' Alzheimer

(30) Priority: 08.05.2003 ES 200301054
(43) Date of publication of application: 17.08.2011
(62) Divisional of application: 08170222.7
(73) Proprietor: Araclón Biotech, S. L., 50008 Zaragoza (ES)
(72) Inventor: Sarasa Barrio, Manuel, 50004 Zaragoza (ES)
(74) Representative: Durán-Corretjer, S.L.P.

(56) References cited:
- EP-A- 1 550 673
- WO-A-00/72880
- WO-A-89/06242
- WO-A-90/12870
- WO-A-99/27944
- WO-A-2004/013172
- ES-A1- 2 201 929
- BARD F ET AL: "Epitope and isotype specificities of antobodies to beta-amyloid peptide for protection against Alzheimer's disease-like neuropathology", PROCEEDINGS OF THE NATIONAL ACADEMY OF SCIENCES OF USA, NATIONAL ACADEMY OF SCIENCE, WASHINGTON, DC, US, vol. 100, no. 4, 18 February 2003 (2003-02-18), pages 2023-2028, XP002982464, ISSN: 0027-8424
- BARD F ET AL: "Peripherally administered antibodies against amyloid beta- peptide enter the central nervous system and reduce pathology in a mouse model of Alzheimer disease", NATURE MEDICINE, NATURE PUBLISHING GROUP, NEW YORK, NY, US, vol. 6, no. 8, 1 August 2000 (2000-08-01), pages 916-919, XP002154518, ISSN: 1078-8956
- ATSUNAGA Y ET AL: "Eight-residue A[beta] peptides inhibit the aggregation and enzymatic activity of A[beta]42", REGULATORY PEPTIDES, ELSEVIER SCIENCE BV, NL, vol. 120, no. 1-3, 15 August 2004 (2004-08-15), pages 227-236, XP009123589, ISSN: 0167-0115
- DOMINGUEZ L ET AL: "Developmental spatiotemporal expression of alzheimer beta - APP isoforms in the chick embryo", INTERNATIONAL JOURNAL OF DEVELOPMENTAL BIOLOGY, UNIVERSITY OF THE BASQUE COUNTRY PRES, LEIOA, ES, vol. 45, no. S1, 1 January 2001 (2001-01-01), pages S73-S74, XP002987616, ISSN: 0214-6282

## Description

The present invention provides means for treatment diseases associated with the presence of amyloid deposits, which include Alzheimer's disease.

### STATE OF THE ART

Certain facts are known about the biochemical and metabolic phenomena associated with the presence of Alzheimer's Disease (AD). Two structural and histopathological changes observed in the brains of those with AD are neurofibrillar tangles (NFT) and amyloid deposits. Intraneuronal neurofibrillar tangles are also present in other neurodegenerative diseases, but the presence of amyloid deposits both in the intraneuronal spaces (neuritic plaques) and close to the microvasculature (vascular plaques) seems to be characteristic of AD. Of these, neuritic plaques seem to be the most common (Price, D.L., and co-workers, Drug Development Research (1985) 5:59-68).

The main component of these amyloid plaques is a peptide of 40-42 amino acids denominated amyloid peptide Aβ4.

The amyloid peptide Aβ4 is a polypeptide that originates from proteolysis from membrane glycoproteins denominated amyloid peptide Aβ4 precursor proteins (βAPP). These proteins, precursors of amyloid peptide, consist of 695 to 770 amino acids, all of them being coded by the same gene.

Two main variants of amyloid peptide Aβ4 have been identified, peptide Aβ40 and Aβ42, containing 40 and 42 amino acids, respectively, which present different tissue distributions in both physiological and pathological conditions. The variant of 42 amino acids is the predominant form in the amyloid plaques located in the brains of patients with AD.

Until present, different possible solutions have been proposed to provide a possible vaccine against AD.

In EP526511 , the administration of homeopathic doses of Aβ to patients with pre-established AD is proposed. However, due to the doses used, the levels of circulating endogenous Aβ in plasma hardly vary, and so no therapeutic benefit is expected.

Schenk et al., (Nature, 1999; 400: 173-177) described immunization of transgenic mice PDAPP with Aβ42, which over expressed human mutant APP, thus preventing the formation of amyloid plaques, neuritic dystrophy and astrogliosis.

In WO9927944 (Schenk D.), a treatment for AD is described by administration to a patient of Aβ42.

A phase III clinical trial in 360 patients diagnosed with medium to moderate AD in 4 European countries and the United States, in which amyloid peptide Aβ42 was used as an antigen, was discontinued after encephalitis was reported in some of the patients (Scrip Daily Online, 25 Feb 2002, S007455320, The Scientist 16 [7]: 22, April 1, 2002).

The problem of using an endogenous protein as a vaccine (or a protein present naturally in the animal that is being vaccinated), as is the case of peptide Aβ42, the organism responds by making antibodies against Aβ42 and against smaller fractions that may also have as yet unknown physiological functions, among some of the possible problems we could mention is the possible development of autoimmune diseases due to the generation of antibodies against the endogenous protein, difficulty in the generation of a immune response due to failure of the immune system for recognizing endogenous antigens, and possible development of an acute inflammatory response.

The present invention is aimed at treatment of Alzheimer's disease and other amyloid diseases by administration of a peptide, of the C-terminus part of Aβ, conjugated with a protein, which in a preferred embodiment of the present invention said protein is the keyhole limpet hemocyanin.

### EXPLANATION OF THE INVENTION

The present invention relates to a vaccine for the treatment of Alzheimer's disease and other related amyloid diseases.

In particular, the present invention is directed to a pharmaceutical composition comprising a peptide with an amino acid sequence comprising SEQ ID No 2, that acts as immunogen to induce antibodies that react with Aβ for use in the treatment of an amyloid disease. The present invention is also directed to a pharmaceutical composition comprising an antibody able to specifically recognize any of the predominant variants of the peptide beta amyloid Aβ40 and Aβ42, for use in the treatment of an amyloid disease, wherein the antibody is obtainable by immunization of mammals or birds, with an amino acid sequence comprising SEQ ID No 2, wherein the amino acid sequence is conjugated to a protein.

According to a preferred embodiment, a vaccine is provided for the treatment of Alzheimer's disease and other related diseases, which overcomes the disadvantages associated with using peptides, proteins or endogenous immunogens.

Examples of other diseases characterized by amyloid deposits are Islandic hereditary syndrome, multiple myeloma, and spongiform encephalitis, including Creutzfeldt-Jakob disease.

The introduction of an immune response can be active such as when an immunogen is administered to induce antibodies that react with Aβ in a patient, or passive, such as when an antibody is administered that reacts by itself with Aβ in a patient.

For the aims of the present invention, the following terms are defined as follows:

The term "related amyloid diseases" includes diseases associated with the accumulation of amyloid which can be restricted to one organ, localized amyloidosis, or spread throughout several organs, systemic amyloidosis. Secondary amyloidosis can be associated with chronic infections (such as, for example, tuberculosis) or chronic inflammation (for example, rheumatoid arthritis), familial Mediterranean fever (FMF) and other types of systemic amyloidosis found in patients in the long-term treatment of hemodialysis. Localized forms of amyloidosis include, but are not limited to, type II diabetes and any other disease related thereto, neurodegenerative diseases with scrapie, bovine spongiform encephalitis, Creutzfeldt-Jakob disease, Alzheimer's disease, cerebral amyloid angiopathy.

The term "passive immunization" is used to relate to the administration of antibodies or fragments thereof to an individual with the intention of conferring immunity on that individual.

In the first aspect, there is described the use of either a peptide that acts as an immunogen or as an antibody, in the preparation of a medication for the treatment of a disease characterized by the accumulation of amyloid deposits. Said methods consist of the induction of an immune response against a peptide component of the amyloid deposits in the patient. Said induction could be active through administration of an immunogen or passive through administration of an antibody or an active fragment or derivative of an antibody.

In a preferred embodiment of the present invention, the disease is Alzheimer's disease.

The medication obtained can be used both in asymptomatic patients such as those who show symptoms of the disease.

The compositions able to provoke an immune response directed against certain components of the amyloid plaques are effective for treatment of diseases related to amyloid deposits. In particular, it is possible to prevent the progress of, reduce the symptoms of and/or reduce the deposition process of amyloid in an individual, when an immunostimulatory dose of a peptide or of an antibody obtained therefrom, is administered to the patient.

In accordance with the present invention, the antibodies are obtained by immunization of mammals or birds by use of a peptide conjugated to a protein as an immunogen.

According to a preferred embodiment of the present invention, the mammals used for immunization can be ruminants, equines, lagomorphs, carnivores, primates, or any other animal that allows adequate quantities of serum to be extracted therefore for antibody. Among the birds used for immunization, we can mention, but in no way limit to, Galliformes, Anseriformes and Columbiformes, among others.

The present invention, provides the use of a peptide with an aminoacid sequence comprising SEQ ID No 2 that acts as an immunogen to induce antibodies that react with Aβ for use in the treatment of an amyloid disease.

The present invention also provides the use of an antibody able to specifically recognizes any of the predominant variants of the beta amyloid peptide, Aβ40 and Aβ42 in the preparation of a medicament for the treatment of an amyloid disease, wherein the antibody is obtainable by immunization of mammals or birds, with an amino acid sequence comprising SEQ ID No 2, wherein the amino acid sequence is conjugated to a protein.

According to the most preferred embodiment of the present invention, the protein used for conjugation with the peptide is keyhole limpet protein.

In this application, the amino acids are abbreviated using the single-letter codes accepted in the field, as indicated below:

| | | |
|---|---|---|
| A = | Ala = | alanine |
| C = | Cys = | cysteine |
| D = | Asp = | aspartic acid |
| E = | Glu = | glutamic acid |
| F = | Phe = | phenylalanine |
| G = | Gly = | glycine |
| H = | His = | histidine |
| I = | Ile = | isoleucine |
| K = | Lys = | lysine |
| L = | Leu = | leucine |
| M = | Met = | methionine |
| N = | Asn = | asparagine |
| P = | Pro = | proline |
| Q = | GIn = | glutamine |
| R = | Arg = | arginine |
| S = | Ser = | serine |
| T = | Thr = | threonine |
| V = | Val = | valine |
| W = | Trp = | tryptophane |
| Y = | Tyr = | tyrosine |

The sequences described in the present invention, and identified as SEQ ID No 2, correspond to the following amino acid sequences:
SEQ ID NO 2 GLMVGGVV

The antibody obtained from the previous peptides is given the codes SAR-3 corresponding to the one shown below:
SEQ ID NO 2 SAR-3

The information relating to identification of the peptide sequences, described in the present invention, that accompaniesy the present document in a computer-readable format, is indicated in the list of sequences that is presented along with this document.

### EXAMPLES

The present invention is illustrated by means of the following examples.

### Example 1. Generation of polyclonal antibodies.

The polyclonal antibody against the peptides conjugated with KLH that was used as immunogen was generated by immunization in New Zealand white rabbits.

The immunogen was injected into two rabbits, with five injections in each rabbit: the first intradermal injection of the peptide-KLH conjugate in PBS and emulsified in complete Freud adjuvant and four more intramuscular injections, as a booster dose on days 14, 28, 49 and 80, of the same peptide-KLH conjugate in PBS but this time emulsified in incomplete Freud adjuvant, with the blood letting done at 90 days to detect the presence of antibodies.

After collecting blood, the serum was separated and pre-purified by desalination and then the antibodies were purified by affinity in a matrix comprising 1.5 ml of EMD-Epoxy activated material (Merck) to which 5 mg of the corresponding peptide was added. The purified fractions were packed in 0.1 % BSA (Sigma) and stored at 4°C, and glycerol 20-50% could be added as a cryoprotector.

### Example 2. WESTERN-BLOT for Aβ

### 1. Electrophoresis

The Laemmli method was used, described in Current Protocols in Molecular Biology, John Wiley and Sons, New York, 1998, modified by improve the separation of small peptides.

The apparatus used was a Miniprotean 3 from Bio-Rad.

A 15% acrylamide gel was used, mixed with the following components:

| STOCK SOLUTIONS | SEPARATING GEL (15%) | STACKING GEL |
|---|---|---|
| 40% acrylamide | 3.75 ml | 500 µl |
| Tris 3 M, pH = 8.45 | 3.3 ml | 250 µl |
| Glycerol | 1.05 ml | - |
| Water | 1.9 ml | 4.2 µl |
| SDS 20% | 50 µl | 18.6 µl |
| APS 10% | 50 µl | 25 µl |
| TEMED | 10 µl | 5 µl |

Initial stock solutions of peptide Aβ40 and 42 of 1 mg/ml were used (dissolved in PBS). The volume necessary was taken of these solutions for each one of the samples and made up to 20 µl with SBLT (SBL + Tris base 2 M). The samples were then boiled for 5 minutes to denature the peptides and eliminate possible proteases.

The center of the cuvette was filled with cathode buffer and the outside with anode buffer, the composition of these buffers being as follows:
Anode buffer
   24.2 g Tris base (0.2 M final concentration)
   Dilute to 1 litre with H₂O
   Adjust of pH 8.9 with concentrated HCl
   Store at 4°C for up to 1 month

Cathode buffer
   12.11 g Tris base (0.1 M final concentration)
   17.92 g tricine (0.1 M final concentration)
   1 g SDS (0.1 % final concentration)
   Dilute to 1 litre with H₂O
   Do not adjust Ph
   Store at 4°C for up to 1 month

Finally, the samples were loaded into the wells: 20 µl/well. Using the Polypeptide Standard Kaleidoscope from Bio-Rad as a marker, migration started at low voltage (30 V), and then the voltage was raised to 100 V, after approximately 1 hour of electrophoresis.

### 2.- MEMBRANE TRANSFER

The proteins separated in the gel were transferred to the PVDF membrane by electroblotting. In the transfer booklets the following were placed

Black side -sponge- 3 Whatmann papers (or filter papers) -gel- - membrane- -3 Whatmann papers- -sponge- -transparent side.

The cuvette was then filled with electroblotting buffer:
Glycine 38 nM
Tris base 50 mM
Methanol 40%

The transfer was done for 2 hours at 200 mA. During the transfer, the buffer was kept stirring with the magnetic stirrer.

### 3- INCUBATION WITH ANTIBODY

The antibody and the powder milk were dissolved in PBS-t (PBS + 0.5% Tween 20), carrying out the washing with PBS-T also.

After the transfer, the surface of the membrane was blocked with 5% solution of powder milk for 1 hour with stirring and at room temperature (RT)

After this, the membrane was washed for 2 x 5 minutes at RT.

Then, it was incubated with primary antibody ( SAR-3 ) for 1 hour at RT at least diluted 1:500 in PBS-T.

The membrane was washed: 3 x 10 minutes at RT. Then, it was incubated with secondary antibody: goat anti-rabbit-HRP for 1 hour at RT (1:10,000 in all cases).

The washing of the membrane was repeated once again: 3 x 10 minutes at RT.

### 4.- DEVELOPMENT

After the last washing, the membrane was incubated with the solution of the chemoluminescence kit, using the ECL kit+Plus from Pharmacia.

The membrane was wrapped in cellophane and exposed to double-emulsion film (Hyperfilm MP from Amersham), for different times of between 30 seconds and 2 minutes.

### Example 3. Immunohistochemistry with SAR-3 antibody in the tissue of human brain.

The sections of tissue were fixed in paraffin following the following steps:
a) fixation in neutral formol at 10%
b) dehydration by successive steps in increasing concentrations of alcohol
c) passes through xylol and paraffin, this latter step in an oven at 60-62°C.
d) carrying out of paraffin blocks, which were cut to 4 microns and mounted in slides.

The sections were then deparaffinized by passing through the following solutions:

| | | |
|---|---|---|
| Xylol | 100% | 10 minutes |
| Xylol | 100% | 10 minutes |
| Ethanol | 100% | 5 minutes |
| Ethanol | 100% | 5 minutes |
| Ethanol | 96% | 5 minutes |
| Ethanol | 90% | 5 minutes |
| Ethanol | 70% | 5 minutes |
| PBS | | 5 minutes x 3 times |

Afterwards, they were treated in the following way:
a) 96% formic acid for 3 minutes in a fume cupboard and with stirring
b) Rapid washing with water
c) Washing in PBS 2 x 5 minutes
d) Block of the endogenous peroxidases for 15 minutes in a solution made up of 70 ml of PBS, 30 ml of methanol and 1 ml of H₂O₂
e) Washing in PBS 3 x 5 minutes
f) Washing in PBS/T (Triton or Tween-20 at 0.5% in PBS) 3 x 5 minutes
g) Block of the non-specific binding with goat serum (Normal Goat Serum) diluted 10:100 in PBS/T for two hours
h) Incubation of the primary antibody all night at 4°C in a moisture chamber:
   Sar-3... Dilution 1:1500 in PBS
i) Washing in PBS/T 3 x 5 minutes
j) Incubation in secondary antibody (anti-rabbit goat) diluted 1:200 in PBS during 45 minutes
k) Washing in PBS 4 x 5 minutes
l) Incubation of ABC (avidin-biotin complex) of Vector Labs at a dilution of 1:100 in PBS/T for 45 minutes in darkness, keeping these conditions until development was complete
m) Washing in PBS 3 x 5 minutes
n) Development in diaminobenzidine (DAB)

The time was controlled empirically under a stereoscopic microscope. For this, first, a washing was done in a solution of Tris-HCl 0.5 M for 10 minutes with shaking, to then continue with incubation with a diaminobenzidine substrate (DAB) diluted in Tris-HCl 0.05M and to which is added 0.5 µ/ml of H₂O₂ at 4°C. Once the reaction was finished, three washes were done in PBS at 4°C for 5 minutes each time and then dehydration in ethanol was done at 70%, 90 % and 100% for 2 minutes each time, passing through xylol for 4 minutes and a further pass through xylol for 2 minutes, until they were mounted with Eukitt for observation under the microscope.

### List of sequences.

NUMBER OF SEQUENCES: 14
INFORMATION ON SEQUENCE 12:
   CHARACTERISTICS OF THE SEQUENCE:
   LENGTH: 8
   TYPE: amino acid
   TYPE OF MOLECULE: peptide
   SOURCE: Chemical synthesis
SEQUENCE DESCRIPTION:

### SEQUENCE LISTING

<110> ARACLON BIOTECH, S.L.
<120> Alzheimer's disease treatment method
<130> EP1513.A.2.div1.div2
<140> EP10012342.1
   <141> 2010-09-30
<160> 4
<170> PatentIn version 3.5
<210> 1
   <211> 8
   <212> PRT
   <213> Artificial Sequence
<220>
   <223> A beta fragment of beta amyloid peptide
<400> 1
   Leu Val Phe Phe Ala Glu Asp Val
   1               5
<210> 2
   <211> 8
   <212> PRT
   <213> Artificial Sequence
<220>
   <223> A beta fragment of beta amyloid peptide
<400> 2
   Gly Leu Met Val Gly Gly Val Val
   1 5
<210> 3
   <211> 10
   <212> PRT
   <213> Artificial Sequence
<220>
   <223> A beta fragment of beta amyloid peptide
<400> 3
   Gly Leu Met Val Gly Gly Val Val Ile Ala
   1 5 10
<210> 4
   <211> 12
   <212> PRT
   <213> Artificial Sequence
<220>
   <223> A beta fragment of beta amyloid peptide
<400> 4
   Arg His Asp Ser Gly Tyr Glu Val His His Gln Lys
   1 5 10

## Claims

1. A pharmaceutical composition comprising a peptide with an amino acid sequence comprising SEQ ID No 2, that acts as immunogen to induce antibodies that react with Aβ for use in the treatment of an amyloid disease.

2. The pharmaceutical composition comprising a peptide with an amino acid sequence comprising SEQ ID No 2 for use according to claim 1, wherein the amyloid disease is Alzheimer's disease or cerebral amyloid angiopathy.

3. A pharmaceutical composition comprising an antibody able to specifically recognize any of the predominant variants of the peptide beta amyloid Aβ40 and Aβ42, for use in the treatment of an amyloid disease, wherein the antibody is obtainable by immunization of mammals or birds, with an amino acid sequence comprising SEQ ID No 2, wherein the amino acid sequence is conjugated to a protein.

4. The pharmaceutical composition comprising an antibody able to specifically recognize any of the predominant variants of the peptide beta amyloid Aβ40 and Aβ42 for use according to claim 3, wherein the protein conjugated to the amino acid sequence used to obtain the antibody by immunization of mammals and birds is keyhole limpet protein (KLH).

## Patentansprüche

1. Pharmazeutische Zusammensetzung aufweisend ein Peptid mit einer Aminosäuresequenz, die SEQ ID NO: 2 aufweist, das als ein Immunogen wirkt, um Antikörper zu induzieren, die mit Aβ reagieren, zur Verwendung bei der Behandlung einer Amyloid-Krankheit.

2. Pharmazeutische Zusammensetzung aufweisend ein Peptid mit einer Aminosäuresequenz, die SEQ ID NO: 2 aufweist, zur Verwendung nach Anspruch 1, wobei die Amyloid-Krankheit Alzheimer-Krankheit oder zerebrale Amyloid-Angiopathie ist.

3. Pharmazeutische Zusammensetzung aufweisend einen Antikörper, der in der Lage ist, spezifisch eine der vorherrschenden Varianten des Peptids β-Amyloid Aβ40 und Aβ42 zu erkennen, zur Verwendung bei der Behandlung einer Amyloid-Krankheit, wobei der Antikörper erhältlich ist durch Immunisierung von Säugetieren oder Vögeln mit einer Aminosäuresequenz, die SEQ ID NO: 2 aufweist, wobei die Aminosäuresequenz an ein Protein konjugiert ist.

4. Pharmazeutische Zusammensetzung aufweisend einen Antikörper, der in der Lage ist, spezifisch eine der vorherrschenden Varianten des Peptids β-Amyloid Aβ40 und Aβ42 zu erkennen, zur Verwendung nach Anspruch 3, wobei das an die Aminosäuresequenz, die zum Erhalten des Antikörpers durch Immunisierung von Säugetieren und Vögeln verwendet wird, konjugierte Protein Schlüsselloch-Napfschneckenprotein (keyhole limpet protein, KLH) ist.

## Revendications

1. Composition pharmaceutique comprenant un peptide ayant une séquence d'acides aminés comprenant SEQ ID No. 2, qui joue le rôle d'immunogène pour induire des anticorps qui réagissent avec Aβ pour une utilisation dans le traitement d'une maladie amyloïde.

2. Composition pharmaceutique comprenant un peptide ayant une séquence d'acides aminés comprenant SEQ ID No. 2, pour une utilisation selon la revendication 1, dans laquelle la maladie amyloïde est la maladie d'Alzheimer ou l'angiopathie amyloïde cérébrale.

3. Composition pharmaceutique comprenant un anticorps capable de reconnaître spécifiquement l'un quelconque des variants prédominants du peptide bêta amyloïde Aβ40 et Aβ42, pour une utilisation dans le traitement d'une maladie amyloïde, dans laquelle l'anticorps peut être obtenu par l'immunisation de mammifères ou d'oiseaux avec une séquence d'acides aminés comprenant SEQ ID No. 2, la séquence d'acides aminés étant conjuguée à une protéine.

4. Composition pharmaceutique comprenant un anticorps capable de reconnaître spécifiquement l'un quelconque des variants prédominants du peptide bêta amyloïde Aβ40 et Aβ42, pour une utilisation selon la revendication 3, dans laquelle la protéine conjuguée à la séquence d'acides aminés utilisée pour obtenir l'anticorps par l'immunisation de mammifères ou d'oiseaux est la protéine d'hémocyanine de patelle (KLH) .
